Europäisches Patentamt

(19) European Patent Office

Office européen des brevets

(11) Publication number : **0 379 846 B1**

(12) **EUROPEAN PATENT SPECIFICATION**

(45) Date of publication of patent specification :
**26.05.93 Bulletin 93/21**

(21) Application number : **89830528.9**

(22) Date of filing : **29.11.89**

(51) Int. Cl.$^5$ : **A61K 7/48,** A61K 35/38,
A61K 35/24, A61K 35/42

(54) **Product for protecting, nourishing and treating the skin.**

(30) Priority : **30.11.88 IT 2279588**

(43) Date of publication of application :
**01.08.90 Bulletin 90/31**

(45) Publication of the grant of the patent :
**26.05.93 Bulletin 93/21**

(84) Designated Contracting States :
**BE CH DE ES FR GB GR LI NL**

(56) References cited :
**WO-A-89/05137
BE-A- 651 601
FR-A- 1 390 184
FR-A- 2 261 755
FR-A- 2 487 197**

(56) References cited :
**FR-A- 2 590 169
FR-M- 1 957
PATENT ABSTRACTS OF JAPAN, vol. 12, no.
299 (C-520)[3146], 15th August 1988**

(73) Proprietor : **PROMO INTERNATIONAL S.r.l.
Via Melloni, 8
I-20129 Milano (IT)**

(72) Inventor : **Imperatori, Diana, Promo
International S.r.l.
Via Melloni, 8
I-20129 Milano (IT)**

(74) Representative : **Cicogna, Franco
Ufficio Internazionale Brevetti Dott.Prof.
Franco Cicogna Via Visconti di Modrone, 14/A
I-20122 Milano (IT)**

## Description

## BACKGROUND OF THE INVENTION

The present invention relates to a product which can be used in the dermatological field as a base element for preparing several cosmetic and curative products.

A lot of skin treating products are commercially available the composition of which includes one or more substances which are considered as useful for treating skin defects generated by different causes, both of the inner and outer type.

These substances,which posses a more or less satisfactory efficacy can be either of chemical,vegetable or animal type.

In particular a great improvement in this field has been achieved based on the study of the mucous layer which continuously covers the mucous membranes of living organisms.

As is known, this protecting layer efficiently protects the mucous cells from outer attacks of physical,chemical and microbiological nature,as well a: against inner attacks,such as,for example,secretions occurring during a digestive process and against toxins which can be formed in the human body because of several reasons.

Notwithstanding this,it has been generally found that the mucous cells,in particular the gastric mucous cells,can survive and properly operate even in a hostile environment,because of the presence of enzymes and hydrochloric acid,since these cells are able of secreting particular biopolymers,forming a mucous layer which protects the cells against the gastric environment attack.

More specifically,the mentioned mucous layer, in addition to favouring and adjusting exchanges,has a protective,lubricating,antibacteric,hydrating and detoxicating activity.

Since several self-evident analogies between the mucous layers of the digestive apparatus and the human skin exist,because of the analogous structure and coating function,it should be obvious to assume that natural colloids (biopolymers) which provide an extraordinary recovering and protecting action on gastric mucous membranes,would have a like action on human skin.

At first,such an extraordinary protective action of the mucous membranes has been mainly ascribed to the presence of particular compounds ( glycoproteins) which are generally called "mucine".Accordingly such a substance has been broadly used in a lot of dermatological products,with consequent greatly interesting results.

However,it has been observed that a very high purification of mucine,instead of providing more active compounds,lowers,the used dose being the same,the skin protecting characteristics.

Based on the above observation and practical experiments,carried out at the cutaneous level,by mucine-based compounds,in association with other biopolymers,in natural ratios,and by purified mucine,it has been found that the hydrating,softening,invigorating,anti-wrinkle action of the purified mucine based product is much smaller.

From the above it can be deduced that the several substances (mucouspolysacarides,proteousglycanes,proteins and nucleic acids) which are present in the full extract,synergically operate,together with the glycoproteins of mucine,to increase the activity thereof.

The document FR-A-2 261 755 discloses a method for preparing a pharmaceutical composition and a pharmaceutical composition prepared thereby, but it does not disclose or address the problem of preparing cosmetic compositions: this document, moreover, does not provide any quantitative analysis for the active substances contained in the pharmaceutical composition, which, accordingly, can not be qualified; this document, finally, does not define any mutual ratio between the biopolymers.

The document PATENT ABSTRACTS OF JAPAN, vol.12, No. 299 (C-250) and JP-0-63 68 512 disclose the use of gastric mucine but not of a biopolymer mixture like that taught by the Applicant it this new biopolymer mixture, moreover, is neither taught by the documents FR-A-1 957 which exclusively discloses proteins and polypeptides for a pharmaceutical use, nor by the document FR-0-2 487 197 which does not provide to use nucleic acids.

## SUMMARY OF THE INVENTION

Accordingly,the aim of the present invention is to provide such a product for protecting,nourishing and treating skin,adapted to be used as a base for preparing a lot of cosmetic products,which includes all of the biopolymers associated with natural mucine.

According to one aspect of the present invention, the above aim is achieved by a product for protecting, nourishing and treating skin, having the features of claim 1.

This mixture,the composition of which can vary within limits of ± 20% is such as to provide very improved results with respect to those which can be obtained by the single components and,in particular, by pure mucine and,more specifically,it has the following activities:

- protective and defensive for the skin and mucous membranes
- anti-wrinkles and anti-ageing
- hydratating
- trophic
- anti-irritating,softening and lenitive

Based on the above mentioned characteristics, the product according to the present invention,which will be called EPIGLYCAN hereinafter,can be advantageously used in the formulation of several products for treating skin and of cosmetic type in general.

Some exemplary embodiments of these products, coming within the spirit of the invention,will be disclosed by way of an indicative but not limitative example hereinbelow.

## ANTI- WRINKLE CREAM

| | |
|---|---|
| EPIGLYCAN | 2 gr |
| AVOCADO OIL | 8 " |
| CETYLSTEARYL ALCOHOL | 6 " |
| OCTYLDODECANOLE | 5 " |
| POLYETHYLENEGLYCOLE STEARATE | 4 " |
| GLYCERINE | 4 " |
| PRESERVATIVES AND PERFUME | as required |
| WATER | balance to 100 gr |

## TONIC PROTECTIVE LOTION

| | |
|---|---|
| EPIGLYCAN | 0.5 gr |
| ROSE WATER | 20 " |
| HAMAMELIS WATER | 20 " |
| MINT WATER | 20 " |
| PRESERVATIVES AND PERFUME | as required |
| WATER | balance to 100 gr |

## HYDRATATING LOTION

| | |
|---|---|
| EPIGLYCAN | 1.5 gr |
| N.M.F. | 5 " |
| GLYCERINE | 5 " |
| MALTITOLE | 3 " |
| PRESERVATIVES AND PERFUME | as required |
| WATER | balance to 100 gr |

## AFTER SHAVE LOTION

| | |
|---|---|
| EPIGLYCAN | 1 gr |
| ALCOHOL 95° | 30 ml |
| PANTENOL | 0.3 gr |
| MENTHOL | 0.05 " |
| ALANTOINE | 0.1 " |
| WATER | balance to 100 gr |

## LENITIVE BARRIER CREAM

| | |
|---|---|
| EPIGLYCAN | 1.5 gr |
| O/W BASE | 10 " |
| PETROLATUM OIL | 30 " |
| FLUID SILICONE | 8 " |
| PRESERVATIVES AND PERFUME | as required |
| WATER | balance to 100 gr |

4

## PERIOCULAR DROPS

| | | |
|---|---|---|
| EPIGLYCAN | 10 | gr |
| HAMAMELIS EXTRACT | 3 | " |
| MALLOW EXTRACT | 3 | " |
| CAMOMILLE EXTRACT | 2 | " |
| PROPYLENEGLYCOLE | 2 | " |
| PRESERVATIVES | as required | |
| WATER | balance to 100 gr | |

## ANTI-AGEING CREAM

| | | |
|---|---|---|
| EPIGLYCAN | 3 | gr |
| E VITAMIN | 0.1 | " |
| BETA CARROTENE | 0.02 | " |
| CARROT OIL | 3 | " |
| YEAST EXTRACT | 1 | " |
| MANNITOL | 3 | " |
| SELF-EMULSIFYING O/W BASE | 10 | gr |
| ALMOND OIL | 3 | " |
| PRESERVATIVES AND PERFUME | as required | |
| WATER | balance to 100 gr | |

The above disclosed products allow several problems to be solved,relating to the protection and treatment of the skin,in a new and very positive way,based on a concrete and serious scientific experiment set.

It should be apparent that the practical method for making the subject cosmetic products,as well as the specific component amounts can vary depending on requirements,provided that in all of the cosmetic products is included,as an essential base,the product according to the present invention,called EPIGLYCAN and which has been hereinabove disclosed.

It should be also apparent that the subject product can also be replaced,either completely or in part,by biopolymer extracts derived from the intestine, respiratory apparatus,rumen,uterus,that is all of the hollow organs of the body which,since they are susceptible of exchanges with the outer environment,and can form aggressive substances,of the chemical,physical and microbiological type must be protected,like the stomach,by a biopolymer based coating layer,for defense and protection purposes.

From the above disclosure it should be apparent that the invention fully achieves the intended aim.

## Claims

1. A product for protecting, nourishing and treating skin, comprising a synergic combination of; biopolymers forming natural mucous substances coating the mucous walls of the digestive apparatus, said biopolymers including biopolymer extracts derived from the intestine, respiratory apparatus, rumen, uterus or other hollow organs of animal bodies, characterized in that said product has the following average formulation
   - glycoproteins relating to mucine        50 %
   - glucosaminoglycans        15 %
   - nucleic acids        10 %
   - extractive collagen proteins        25 %
   and that said formulation may vary within formulation limits of ± 20%.

2. A product according to Claim 1, characterized in that said product is adapted to be included, as an essential base, into the composition of a number of different products, for treating skin and of the cosmetic type in general, in order to provide at least the following specific actions:
   - protective and defensive for the skin and mucous tissues
   - anti-wrinkles and anti-ageing
   - hydrating
   - trophic
   - anti-irritating, softening and lenitive.

## Patentansprüche

1. Mittel zum Schützen, Ernähren und Behandeln der Haut, mit einer synergischen Kombination von Polymere, die die natürlichen Schleimstoffe bilden, die die Schleimwände des Verdauungsapparates verkleiden, wobei diese Biopolymere solchen biopolymerischen Extrakte einschliessen, die aus dem Darm, dem Atmungsapparat, dem Magen, der Gebärmutter oder anderen hohlen Organe der Tierkörper herkommen, dadurch gekennzeichnet, dass dies Mittel die folgende mittlere Formel besitzt :
   - Glykoproteine betr. Mucine        50%
   - Glukoseaminoglykane        15%
   - Nucleinsäure        10%
   - extraktive Kollageneproteine        25%
   und dass diese Formel innerhalb Formelgrenzen von ± 20% ändern kann.

2. Mittel nach Anspruch 1, dadurch gekennzeichnet, dass dies Mittel eine Zusammensetzung kosmetischer Art von mehreren verschiedenen Mitteln zur Behandlung der Haut als essentialer Grundstoff zu angehören geeignet ist, somit wenigstens die folgenden spezifischen Wirkungen gebildet werden :
   - eine Schutzwirkung für die Haut und den Schleimstoffe
   - eine Wirkung gegen den Runzen un das Altern
   - eine feuchtigkeitsspendende Wirkung
   - eine trophische Wirkung
   - eine beruhigende und reizlindernde Wirkung gegen den Entzündungen.

## Revendications

1. Produit pour protéger, nourrir et traiter la peau, comprenant une combination synergique de biopolymères formant des substances muqueuses naturelles recouvrant les parois muqueuses de l'appareil digestif, lesdits biopolymères comprenant des extraits dérivés de l'intestin, de l'appareil respiratoire, du rumen, de l'utérus ou d'autres organes creux des corps des animaux,
caractérisé en ce que ledit produit présente la formulation moyenne suivante
   - glycoprotéines concernant mucine        50%
   - glucoseaminoglycans        15%

- acides nucléiques          10%
- protéines collagéniques extractives          25%

et en ce que ladite formulation peut varier dans les limites de la formulation de ± 20%.

2. Produit selon la revendication 1, caractérisé en ce que ledit produit est adapté pour être inclus, comme base essentielle, dans la composition de plusieurs produits différents pour le traitement de la peau et du type cosmétique en général, en vue de produire au moins les actions spécifiques suivantes:
   - une action de protection et de défense pour la peau et les tissus muqueux;
   - une action anti-rides et anti-age;
   - une action hydratante;
   - une action trophique;
   - une action anti-irritation, adoucissante et lénitive.